# EUROPÄISCHE PATENTSCHRIFT

(11) **EP 1 194 424 B1**
(45) Veröffentlichungstag und Bekanntmachung des Hinweises auf die Patenterteilung: **06.10.2004**
(21) Anmeldenummer: 01945257.2
(22) Anmeldetag: 06.06.2001
(51) Int. Cl.: C07D 311/92, G02B 5/23

(54) **PHOTOCHROME PYRANDERIVATE**
PHOTOCHROMIC PYRAN DERIVATIVES
DERIVES DE PYRANE PHOTOCHROMIQUES

(30) Priorität: 07.06.2000 DE 10027763
(43) Veröffentlichungstag der Anmeldung: 10.04.2002
(73) Patentinhaber: Rodenstock GmbH, 80469 München (DE)
(72) Erfinder: MANN, Claudia, 80634 München (DE); WEIGAND, Udo, 80638 München (DE); MELZIG, Manfred, 82234 Wessling (DE)
(74) Vertreter: Müller-Boré & Partner Patentanwälte
(86) Internationale Anmeldenummer: PCT/EP2001/006392
(87) Internationale Veröffentlichungsnummer: WO 2001/094336

(56) Entgegenhaltungen:
- EP-A- 1 054 010
- EP-A- 1 116 723
- DE-A- 19 902 771

## Beschreibung

Die vorliegende Erfindung betrifft spezifische photochrome Pyranderivate sowie deren Verwendung in Kunststoffen aller Art, insbesondere für ophthalmische Zwecke. Insbesondere betrifft die vorliegende Erfindung von Naphthopyranen abgeleitete Spiroverbindungen mit Fluorenstruktur, sogenannte Spirofluorenopyrane. Durch die Einführung von jeweils nur einer stark elektronenschiebenden oder -ziehenden Gruppe an spezifische Positionen in der Peripherie des Pyranfarbstoffs werden erfindungsgemäß photochrome Verbindungen erhalten, die sich in der angeregten Form durch ein erweitertes Farbenspektrum, d.h. durch eine hypsochrome bzw. bathochrome Verschiebung des längstwelligen Absorptionsmaximums bei gleichzeitig vergleichbar guten Aufhellungsgeschwindigkeiten und gutem Verhalten im Lebensdauertest auszeichnen.

Es sind verschiedene Farbstoffklassen bekannt, die bei Bestrahlung mit Licht bestimmter Wellenlängen, insbesondere Sonnenstrahlen, reversibel ihre Farbe wechseln. Dies rührt daher, daß diese Farbstoffmoleküle durch Energiezufuhr in Form von Licht in einen angeregten farbigen Zustand übergehen, den sie bei Unterbrechung der Energiezufuhr wieder verlassen, wodurch sie in ihren farblosen oder zumindest kaum gefärbten Normalzustand zurückkehren. Zu diesen photochromen Farbstoffen gehören beispielsweise die Naphthopyrane, die im Stand der Technik mit verschiedenen Substituenten bereits beschrieben wurden.

Pyrane, speziell Naphthopyrane und von diesen abgeleitete größere Ringsysteme, sind photochrome Verbindungen, die bis heute Gegenstand intensiver Untersuchungen sind. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen.

Die im Stand der Technik bekannten, von 1-Naphtholen abgeleiteten 2H-Naphthopyrane und ihre davon durch Annellierung abgeleiteten höheren analogen Derivate sind jedoch mit Nachteilen verbunden, die bei deren Verwendung in Sonnenschutzgläsern den Tragekomfort des Brillenträgers wesentlich beeinträchtigen. Die im Stand der Technik bekannten Farbstoffe weisen dabei zum einen häufig eine nicht ausreichend langwellige Absorption im angeregten wie im nicht angeregten Zustand auf. Dies führt auch bei Kombinationen mit weiteren photochromen Farbstoffen zu Problemen. Zum anderen liegt häufig auch eine zu hohe Temperaturempfindlichkeit bezüglich der Eindunkelung vor, wobei gleichzeitig eine zu langsame Aufhellung eintritt. Darüberhinaus besitzen die beschriebenen Farbstoffe eine ungenügende Lebensdauer. Infolgedessen zeigen derartige Sonnenschutzgläser eine unzureichende Haltbarkeit. Letzteres macht sich in schnell nachlassender Leistung und/oder starker Vergilbung bemerkbar.

Durch die Einführung eines Spirofluorengerüsts, wie in DE 198 24 278 bzw, DE 199 02 771 beschrieben, wurden photochrome Verbindungen bereitgestellt, die dahingehend verbesserte Eigenschaften aufweisen. Allerdings liegt deren längstwelliges Absorptionsmaximum der angeregten Form in einem sehr engen Wellenlängenbereich von 570 nm bis 620 nm. Damit sind beispielsweise grüne Sonnenschutzgläser nur durch Gemische verschiedener photochromer Farbstoffe erhältlich. Da bei derartigen Farbstoffgemischen aber die Kinetik der einzelnen photochromen Farbstoffe nicht völlig aufeinander abstimmbar bzw. angleichbar ist, ist das Auftreten von Farbunterschieden während der Eindunkelung und Aufhellung unvermeidlich.

EP-A-1 116 723, welches ein Dokument gemäß Artikel 54(3)(4) EPÜ ist, beschreibt spezifische spirogebundene Chromen-bzw. indeno-annellierte Naphthopyranverbindungen.

Somit liegt der vorliegenden Erfindung die Aufgabe zugrunde, neue photochrome Pyrane bereitzustellen, die sich gegenüber den im Stand der Technik verfügbaren Verbindungen, insbesondere solchen, wie in DE 198 24 278 bzw. DE 199 02 771 beschrieben, in der angeregten Form durch ein breiteres Farbenspektrum auszeichnen sollen, gleichzeitig aber vergleichbar gute Eigenschaften, wie schnelle Kinetik, insbesondere schnelle Aufhellungsgeschwindigkeit, sowie ausgezeichnetes Verhalten im Lebensdauertest, aufweisen sollen. Beispielsweise sollen damit in alleiniger Verwendung grüne Sonnenschutzgläser erhältlich sein.

Diese Aufgabe wird durch die in den Ansprüchen gekennzeichneten Gegenstände gelöst.

Insbesondere werden photochrome Pyrane mit der allgemeinen Formel (I) bereitgestellt: 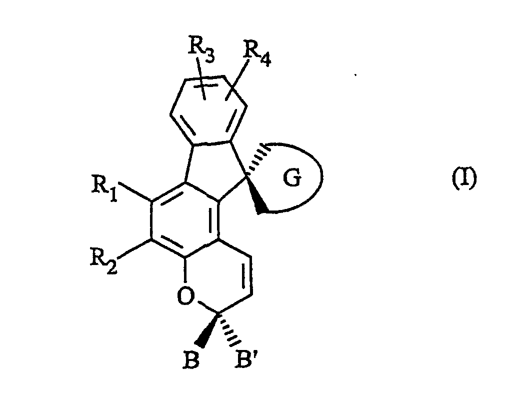 worin die Reste R₁, R₂, R₃ und R₄ jeweils unabhängig voneinander einen Substituenten darstellen, ausgewählt aus der Gruppe α, bestehend aus den Untergruppen
- A:: bestehend aus einem Wasserstoffatom, einem (C₁-C₆)-Alkylrest, einem (C₁-C₆)-Alkoxyrest, einem (C₃-C₇)-Cycloalkylrest, der ein oder mehrere Heteroatome aufweisen kann, einem Arylrest, einem Heteroarylrest, einem Benzylrest, einer Hydroxygruppe, Brom, Chlor und Fluor; und
- A':: wonach die Reste R₁ und R₂ bzw. R₃ und R₄ jeweils eine an den aromatischen Ring gebundene -O-(CH₂)ₙ-O- Gruppe mit n = 1 oder 2 bilden,
- A":: bestehend aus stark elektronenziehenden Gruppen, ausgewählt aus -CF₃, -NO₂, -CN und -SO₂R⁵, wobei R⁵ aus der Untergruppe A ausgewählt ist, und stark elektronenliefernden Gruppen, ausgewählt aus einem Thio(C₁-C₆)-alkylrest, einer Thiophenylgruppe, einer Thiobenzylgruppe, einer Thiomorpholingruppe, einer Morpholingruppe, einer Piperidingruppe, einer Azacycloheptangruppe, einer Piperazingruppe, einer Pyrrolidingruppe, einer Pyrazolidingruppe und einer un-, mono- oder disubstituierten Aminogruppe, wobei die Aminsubstituenten aus einem (C₁-C₆)-Alkylrest, einem (C₃-C₇)-Cycloalkylrest, einem Phenylrest oder einem Benzylrest ausgewählt sein können,
mit der Maßgabe, daß R¹ und R² entweder direkt an die Benzopyraneinheit gemäß der Formel (I) oder über ein daran annelliertes aromatisches oder heteroaromatisches Ringsystem unter Bildung einer entsprechenden Naphthopyraneinheit gebunden sind,
und mit der weiteren Maßgabe, daß innerhalb der Reste R₁ bzw. R₂ und/oder innerhalb der Reste R₃ bzw. R₄ jeweils nur eine stark elektronenziehende Gruppe oder stark elektronenliefernde Gruppe eingeführt ist, ausgewählt aus der Untergruppe A",
die Struktureinheit G unter Einbeziehen des zentralen Spirokohlenstoffatoms ein gesättigtes und/oder ungesättigtes Ringglied mit 5 bis 8 Kohlenstoffatomen darstellt, von denen maximal eines durch ein Heteroatom, ausgewählt aus der Gruppe, bestehend aus O, S und NR₅, ersetzt sein kann, wobei der Rest R₅ wie oben definiert ist, wobei an das Ringglied mindestens ein aromatisches oder heteroaromatisches Ringsystem annelliert ist, wobei das Ringsystem ausgewählt ist aus der Gruppe E, bestehend aus Benzol, Naphthalin, Phenanthren, Pyridin, Chinolin, Furan, Thiophen, Pyrrol, Benzofuran, Benzothiophen, Indol und Carbazol, und das Ringsystem einen oder mehrere Substituenten aus der Gruppe α, vorzugsweise aus der Untergruppe A, aufweisen kann;
B und B' unabhängig voneinander aus einer der folgenden Gruppen a), b), c) oder d) ausgewählt sind, wobei sie
a) mono-, di- und trisubstituierte Arylreste sind, wobei der Arylrest Phenyl oder Naphthyl ist;
b), unsubstituierte, mono- und disubstituierte Heteroarylreste sind, wobei der Heteroarylrest Pyridyl, Furanyl, Benzofuran-2-yl, Benzofuran-3-yl, Thienyl, Benzothien-2-yl, Benzothien-3-yl oder Julodinyl ist;
   wobei die Substituenten der Aryl- oder Heteroarylreste in a) und b) solche sind, ausgewählt aus einer Gruppe, bestehend aus Hydroxy, Amino, Mono-(C₁-C₆)-alkylamino, Di-(C₁-C₆)-alkylamino, am Aromaten un-, mono- oder disubstituiertem Mono- und Diphenylamino, Piperidinyl, Morpholinyl, Carbazolyl, un-, mono- und disubstituiertem Pyrryl, (C₁-C₆)-Alkyl, (C₁-C₆)-Alkoxy, Brom, Chlor und Fluor, wobei die vorgenannten aromatischen und heteroaromatischen Ringsysteme mit (C₁-C₆)-Alkyl, (C₁-C₆)-Alkoxy, Brom, Chlor und Fluor substituiert sein können;
c) Struktureinheiten mit den folgenden Formeln (V) und (W) sind: 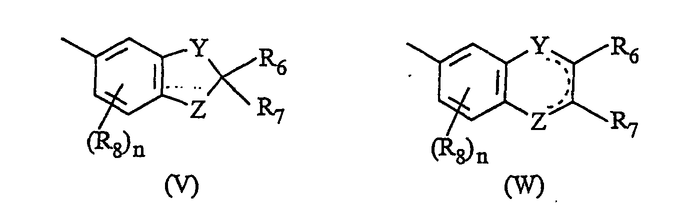 worin
   Y und Z unabhängig voneinander O, S, CH, CH₂ oder NR₉ sind, wobei der Rest R₉ ausgewählt ist aus der Gruppe D, bestehend aus (C₁-C₆)-Alkyl, (C₁-C₆)-Acyl, Phenyl und Wasserstoff, die Reste R₆ und R₇ unabhängig voneinander Wasserstoff und/oder einen (C₁-C₆)-Alkylrest darstellen und der Rest R₈ ein Substituent aus der Untergruppe A ist, wobei n 0, 1, 2 oder 3 ist, mit der Maßgabe, daß, wenn Y in der Formel (V) NR₉ ist, Z Kohlenstoff ist,
   oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorensubstituenten aus der Untergruppe A ausgewählt sind.

Vorzugsweise ist die Struktureinheit G in der vorstehenden Formel (I) unter Einbeziehen des zentralen Spirokohlenstoffatoms eine substituierte oder unsubstituierte Fluoren-, Xanthen-, Thioxanthen-, Phenanthrenyl- oder Dihydroanthracen-Einheit.

Bevorzugte photochrome Verbindungen sind 2H-Naphtho[1,2-b]pyrane mit der nachfolgenden allgemeinen Formel (II): 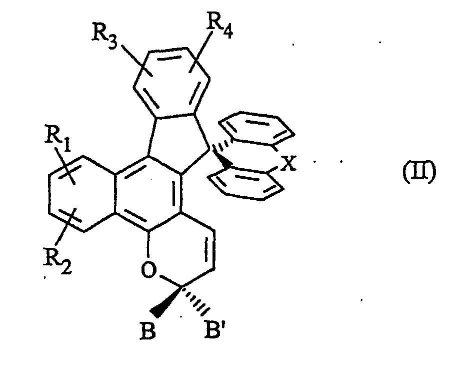 worin R₁, R₂, R₃ und R₄ wie vorgenannt definiert sind und X für -CR'₂-, -CR'=CR'-, -O-, -S- oder eine σ-Einfachbindung steht, wobei der Rest R' unabhängig voneinander aus der Untergruppe A ausgewählt ist, mit der vorstehenden Maßgabe, daß innerhalb der Reste R₁ bzw. R₂ und/oder innerhalb der Reste R₃ bzw. R₄ jeweils nur einer eine stark elektronenziehende Gruppe oder stark elektronenliefernde Gruppe ist, ausgewählt aus der Untergruppe A". Vorzugsweise sind die Reste R₁ und R₂ in 6- bzw. 7-Stellung der Naphthopyraneinheit gemäß vorstehender Formel (II) gebunden.

In einer bevorzugten Ausführungsform ist innerhalb der Reste R₁ bzw. R₂ und/oder innerhalb der Reste R₃ bzw. R₄ gemäß der vorstehenden Formeln (I) bzw. (II) jeweils nur einer eine stark elektronenliefernde Gruppe, wobei als eine stark elektronenliefernde Gruppe eine Morpholingruppe, eine Piperidingruppe, eine Azacycloheptangruppe, eine Piperazingruppe, eine Pyrrolidingruppe, eine Pyrazolidingruppe und eine un-, mono- oder disubstituierte Aminogruppe, wobei die Aminsubstituenten aus einem (C₁-C₆)-Alkylrest, einem (C₃-C₇)-Cycloalkylrest, einem Phenylrest oder einem Benzylrest ausgewählt sein können, bevorzugt sind. In einer besonders bevorzugten Ausführungsform ist innerhalb der Reste R₁ bzw. R₂ und innerhalb der Reste R₃ bzw. R₄ jeweils nur einer eine stark elektronenliefernde Gruppe, d.h. die erfindungsgmäße Verbindung weist in der Peripherie der Indenobenzopyran- bzw. Indenonaphthopyraneinheit gemäß der Formeln (I) bzw. (II) räumlich getrennt voneinander zwei elektronenenliefernde Substituenten auf.

Vorzugsweise sind in den vorstehend aufgeführten Formeln (I) oder (II) B und B' unabhängig voneinander mono-, di- und trisubstituierte Arylreste, wobei der Arylrest jeweils ein Phenylrest oder ein Naphthylrest ist. Die in der Untergruppe A angeführten Aryl-. bzw. Heteroarylreste können insbesondere Phenyl, Naphthyl, Pyrrol, Pyrazol, Imidazol, Imidazolin, Pyridin, Pyrimidin, Pyrazin, Indol, Indazol, Chinolin, Isochinolin, Purin, Pyrimidin, Phenanthridin, Acridin, Phenazin, Carbazol, Thiazol oder Oxazol sein.

Besonders bevorzugte Verbindungen gemäß der vorliegenden Erfindung sind:
Spiro-9-fluoren-13'-[6-(N-morpholinyl)-3-[4-(N-morpholinyl)phenyl]-3-phenylindeno[2,1-f]-naphtho[1,2-b]pyran];
Spiro-9-fluoren-13'-[6-dimethylamino-3-[4-(N-morpholinyl)phenyl]-3-phenylindeno[2,1-f]-naphtho[1,2-b]pyran] und
Spiro-9-fluoren-13'-[6,11-bis(N-morpholinyl)-3-[4-(N-morpholinyl)phenyl]-3-phenylindeno[2,1-f]-naphtbo[1,2-b]pyran].

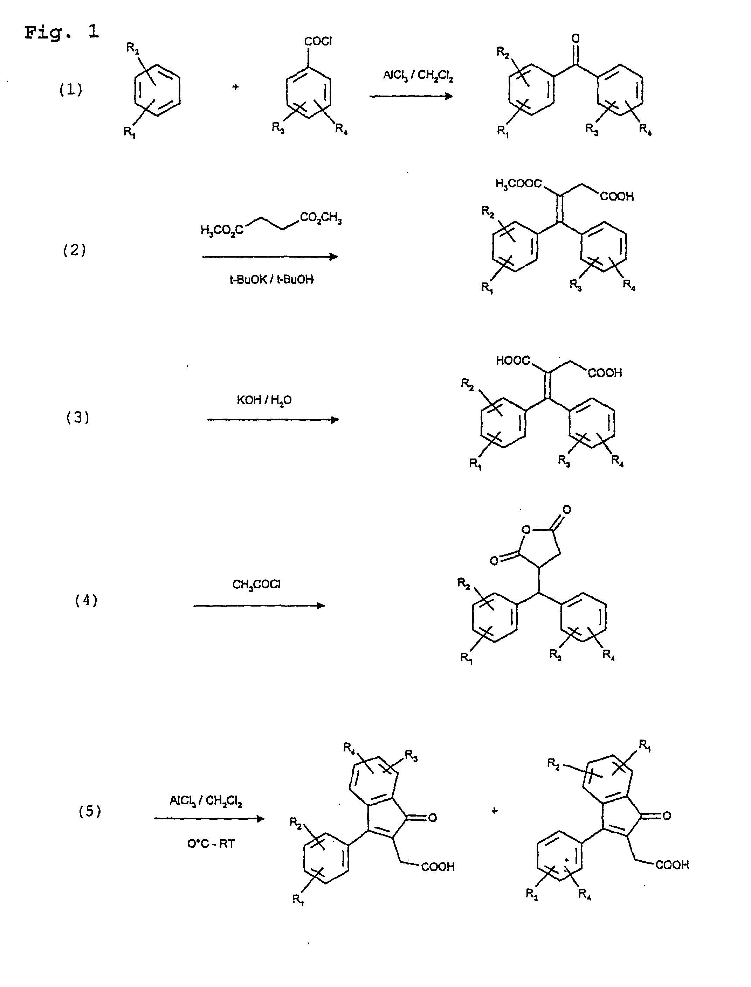

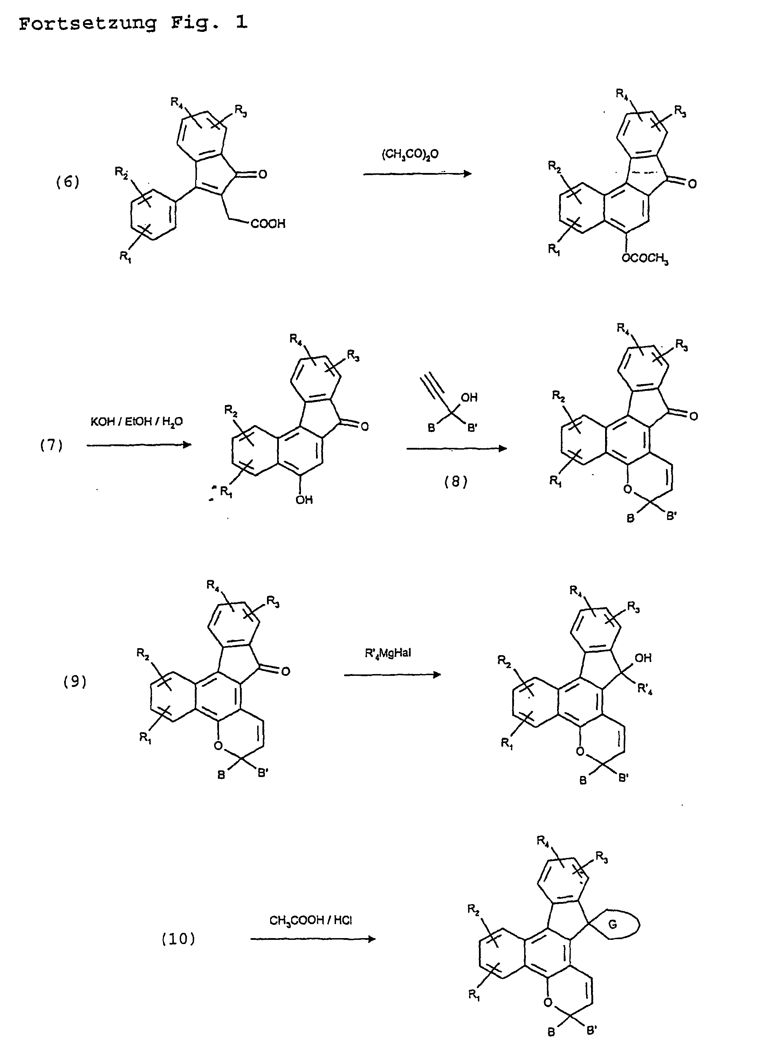

Fig. 1 zeigt schematisch einen Syntheseweg zur Herstellung beispielhafter erfindungsgemäßer photochromer Verbindungen.

Durch die Einführung von jeweils nur einer stark elektronenschiebenden oder - ziehenden Gruppe, ausgewählt aus der Untergruppe A", bezüglich der Reste R₁ bzw. R₂ und/oder der Reste R₃ bzw. R₄ werden photochrome Verbindungen erhalten, die sich gegenüber den im Stand der Technik verfügbaren Verbindungen, insbesondere solchen, wie in DE 198 24 278 bzw. DE 199 02 771 beschrieben, in der angeregten Form durch ein erweitertes Farbenspektrum, d.h. durch eine hypsochrome bzw. bathochrome Verschiebung des längstwelligen Absorptionsmaximums bei gleichzeitig vergleichbar guten Aufhellungsgeschwindigkeiten und gutem Verhalten im Lebensdauertest auszeichnen. Insbesondere das Einführen von jeweils nur einer stark elektronenliefernden Gruppe wie einer Morpholingruppe, einer Piperidingruppe, einer Azacycloheptangruppe, einer Piperazingruppe, einer Pyrrolidingruppe, einer Pyrazolidingruppe oder einer un-, mono- oder disubstituierten Aminogruppe innerhalb der Reste R₁ bzw. R₂ und/oder innerhalb der Reste R₃ bzw. R₄ liefert photochrome Farbstoffe, die in den für Brillengläsern üblicherweise verwendeten Kunststoffen in alleiniger Verwendung, d.h. als einzelner Farbstoff, einen für den Anwender kosmetisch ansprechenden blaugrünen bis grünen Farbeindruck ergeben.

Ein kosmetisch besonders ansprechender graugrüner Farbeindruck kann durch die zusätzliche Verwendung von erfindungsgemäßen Verbindungen mit elektronenziehenden Substituenten erreicht werden. Die Kinetik wird durch die unterschiedliche Substitution, die jeweils in einer vom photochromen Reaktionszentrum sehr weit entfernten Position vorliegt, kaum beeinflusst, sie wird durch die Einheit "G" sowie die Substituenten B und B' dominiert. Bei den erfindungsgemäßen Verbindungen mit elektronenschiebenden Substituenten lässt die bathochrome Verschiebung des längstwelligen Absorptionsmaximums der angeregten Form im Bereich um 500-550 nm ein relatives Absorptionsloch entstehen. Der grüne Farbeindruck entsteht durch die Transmission gerade dieses Wellenlängenbereichs. Ein geringer Zusatz einer erfindungsgemäßen Verbindung mit hypsochrom verschobener Absorption (um ca. 550 nm), d.h. einer erfindungsgemäßen Verbindung mit elektronenziehenden Substituenten neutralisiert den Farbeindruck in Richtung graugrün. Da das Maximum der Empfindlichkeit des menschlichen Auges für das Tagsehen (zugleich Farbsehen) bei 550 nm liegt, ergibt dieser Zusatz zugleich eine deutliche Verringerung der Transmission nach V_{λ}, d.h. eine höhere Absorption. Diese wird aber gerade von Sonnenschutzgläsem erwartet bzw. gefordert.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei kann ein Farbstoff gemäß der vorliegenden Erfindung in alleiniger Verwendung eingesetzt werden. Jedoch ist es im Rahmen der vorliegenden Erfindung auch vorgesehen, eine oder mehrere der erfindungsgemäßen Verbindungen mit anderen photochromen Farbstoffen zu mischen. Beispielsweise können die photochromen Farbstoffe gemäß der vorliegenden Erfindung in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Pyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Pyranfarbstoffe durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfaßt beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Pyranfarbstoffe beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

Die erfindungsgemäßen photochromen Pyrane mit der allgemeinen Formel (I), insbesondere 2H-Naphtho[1,2-b]pyrane bzw. von Naphthopyranen abgeleitete Spiroverbindungen mit Fluorenstruktur, sogenannte Spirofluorenopyrane, mit der allgemeinen Formel (II) lassen sich beispielsweise nach dem in Figur 1 gezeigten Reaktionsschema herstellen. Die als Ausgangsmaterial verwendeten substituierten bzw. unsubstituierten Benzophenone sind entweder im Handel erhältlich oder durch herkömmliche Friedl-Crafts-Acylierung (Schritt(1 )) zugänglich. Anschließende Stobbe-Kondensation mittels Bernsteinsäurediester in Schritt (2) führt zu einem Halbester, der, sofern die beiden über die Ketogruppe verbundenen aromatischen Ringe nicht identisch sind, üblicherweise als Isomerengemisch anfällt. Dieses Halbestergemisch wird anschließend in Schritt (3) mit wäßrigem Alkali verseift, wodurch ein Isomerengemisch der entsprechenden Disäuren erhalten wird. Durch Erwärmen mit Acetylchlorid in Schritt (4) wird dann unter Wasserabspaltung ein Isomerengemisch der entsprechenden cyclischen Anhydride erhalten. In Schritt (5) wird dieses Gemisch mit AlCl₃ umgesetzt, wobei eine intramolekulare 5-Ring-Cyclisierung erfolgt. Im allgemeinen kristallisieren die in Schritt (5) erhaltenen Verbindungen sehr gut und können dank ihrer unterschiedlichen Löslichkeit in Benzol leicht in die beiden Isomeren getrennt werden. In dem Schritt (6) erfolgt bei Umsetzung mit Ac₂O eine 6-Ring-Cyclisierung unter Erhalten einer entsprechenden Esterverbindung, die in Schritt (7) anschließend alkalisch verseift wird. Diese wird anschließend in Schritt (8) mit 2-Propin-1-ol-Derivaten zu Indeno-annellierten Naphthopyran-Derivaten umgesetzt. Dieser Schritt sowie die beiden anschließenden Schritte erfolgen analog zu den Umsetzungen mit isomeren Hydroxyfluorenon-Derivaten, wie in EP-A-0 987 260 beschrieben. In Schritt (9) erfolgt die Umsetzung mit geeigneten Grignard-Reagenzien, die sich in Schritt (10) zu den erfindungsgemäßen Verbindungen cyclisieren lassen; vgl. jeweils Fig. 1.

## Patentansprüche

1. Photochrome Pyrane mit der allgemeinen Formel (I): 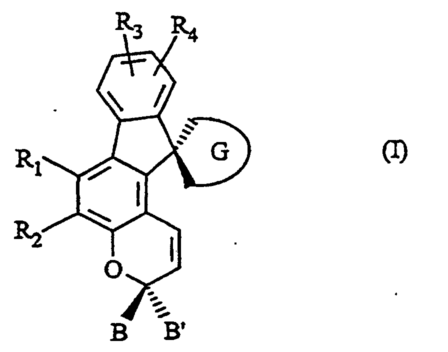 worin die Reste R₁, R₂, R₃ und R₄ jeweils unabhängig voneinander einen Substituenten darstellen, ausgewählt aus der Gruppe α, bestehend aus den Untergruppen
A: bestehend aus einem Wasserstoffatom, einem (C₁-C₆)-Alkylrest, einem (C₁-C₆)-Alkoxyrest, einem (C₃-C₇)-Cycloalkylrest, der ein oder mehrere Heteroatome aufweisen kann, einem Arylrest, einem Heteroarylrest, einem Benzylrest, einer Hydroxygruppe, Brom, Chlor und Fluor, und
A': wonach die Reste R₁ und R₂ bzw. R₃ und R₄ jeweils eine an den aromatischen Ring gebundene -O-(CH₂)ₙ-O- Gruppe mit n = 1 oder 2 bilden,
A": bestehend aus stark elektronenziehenden Gruppen, ausgewählt aus -CF₃, -NO₂, -CN und -SO₂R⁵, wobei R⁵ aus der Untergruppe A ausgewählt ist, und stark elektronenliefernden Gruppen, ausgewählt aus einem Thio(C₁-C₆)-alkylrest, einer Thiophenylgruppe, einer Thiobenrylgruppe, einer Thiomorpholingruppe, einer Morpholingruppe, einer Piperidingruppe, einer Azacycloheptangruppe, einer Piperazingruppe, einer Pyrrolidingruppe, einer Pyrazolidingruppe und einer un-, mono- oder disubstituierten Aminogruppe, wobei die Aminsubstituenten aus einem (C₁-C₆)-Alkylrest, einem (C₃-C₇)-Cycloalkylrest, einem Phenylrest oder einem Benzylrest ausgewählt sein können,
mit der Maßgabe, daß R¹ und R² entweder direkt an die Benzopyraneinheit gemäß der Formel (I) oder über ein daran annelliertes aromatisches oder heteroaromatisches Ringsystem unter Bildung einer entsprechenden Naphthopyraneinheit gebunden sind,
und mit der weiteren Maßgabe, daß innerhalb der Reste R₁ bzw. R₂ und/oder innerhalb der Reste R₃ bzw. R₄ jeweils nur eine stark elektronenziehende Gruppe oder stark elektronenliefernde Gruppe eingeführt ist, ausgewählt aus der Untergruppe A",
die Struktureinheit G unter Einbeziehen des zentralen Spirokohlenstoffatoms ein gesättigtes und/oder ungesättigtes Ringglied mit 5 bis 8 Kohlenstoffatomen darstellt, von denen maximal eines durch ein Heteroatom, ausgewählt aus der Gruppe, bestehend aus O, S und NR₅, ersetzt sein kann, wobei der Rest R₅ wie oben definiert ist, wobei an das Ringglied mindestens ein aromatisches oder heteroaromatisches Ringsystem annelliert ist, wobei das Ringsystem ausgewählt ist aus der Gruppe E, bestehend aus Benzol, Naphthalin, Phenanthren, Pyridin, Chinolin, Furan, Thiophen, Pyrrol, Benzofuran, Benzothiophen, Indol und Carbazol, und das Ringsystem einen oder mehrere Substituenten aus der Gruppe α aufweisen kann; B und B' unabhängig voneinander aus einer der folgenden Gruppen a), b), c) oder d) ausgewählt sind, wobei sie
a) mono-, di- und trisubstituierte Arylreste sind, wobei der Arylrest Phenyl oder Naphthyl ist;
b) unsubstituierte, mono- und disubstituierte Heteroarylreste sind, wobei der Heteroarylrest Pyridyl, Furanyl, Benzofuran-2-yl, Benzofuran-3-yl, Thienyl, Benzothien-2-yl, Benzothien-3-yl oder Julolidinyl ist;
wobei die Substituenten der Aryl- oder Heteroarylreste in a) und b) solche sind, ausgewählt aus einer Gruppe, bestehend aus Hydroxy, Amino, Mono-(C₁-C₆)-alkylamino, Di-(C₁-C₆)-alkylamino, am Aromaten un-, mono- oder disubstituiertem Mono- und Diphenylamino, Piperidinyl, Morpholinyl, Carbazolyl, un-, mono- und disubstituiertem Pyrryl, (C₁-C₆)-Alkyl, (C₁-C₆)-Alkoxy, Brom, Chlor und Fluor, wobei die vorgenannten aromatischen und heteroaromatischen Ringsysteme mit (C₁-C₆)-Alkyl, (C₁-C₆)-Alkoxy, Brom, Chlor und Fluor substituiert sein können;
c) Struktureinheiten mit den folgenden Formeln (V) und (W) sind: 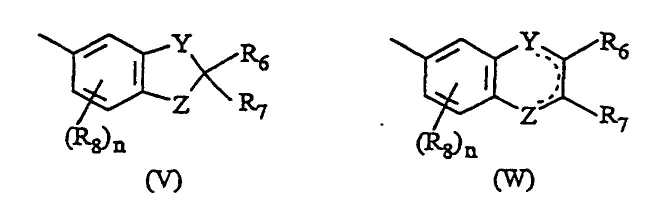 worin
Y und Z unabhängig voneinander O, S, CH, CH₂ oder NR₉ sind, wobei der Rest R₉ ausgewählt ist aus der Gruppe D, bestehend aus (C₁-C₆)-Alkyl, (C₁-C₆)-Acyl, Phenyl und Wasserstoff, die Reste R₆ und R₇ unabhängig voneinander Wasserstoff und/oder einen (C₁-C₆)-Alkylrest darstellen und der Rest R₈ ein Substituent aus der Untergruppe A ist, wobei n 0, 1, 2 oder 3 ist, mit der Maßgabe, daß, wenn Y in der Formel (V) NR₉ ist, Z Kohlenstoff ist,
oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorensubstituenten aus der Untergruppe A ausgewählt sind.

2. Photochrome Pyrane nach Anspruch 1, wobei die Struktureinheit G in der Formel (I) unter Einbeziehen des zentralen Spirokohlenstoffatoms eine substituierte oder unsubstituierte Fluoren-, Xanthen-, Thioxanthen-, Phenanthrenyl- oder Dihydroanthracen-Einheit ist.

3. Photochrome Pyrane nach Anspruch 1 oder 2, welche die allgemeine Formel (II) aufweisen: 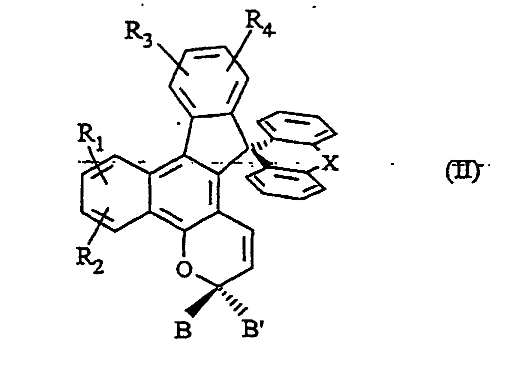 worin R₁, R₂, R₃ und R₄ wie vorgenannt definiert sind und X für -CR'₂-, -CR'=CR'-, -O-, -S- oder eine σ-Einfachbindung steht, wobei der Rest R' unabhängig voneinander aus der Untergruppe A ausgewählt ist, mit der vorstehenden Maßgabe, daß innerhalb der Reste R₁ bzw. R₂ und/oder innerhalb der Reste R₃ bzw. R₄ jeweils nur einer eine stark elektronenziehende Gruppe oder stark elektronenliefernde Gruppe ist, ausgewählt aus der Untergruppe A".

4. Photochrome Pyrane nach Anspruch 3, wobei die Reste R₁ bzw. R₂ in 6-bzw. 7-Stellung der Naphthopyraneinheit gemäß vorstehender Formel (II) gebunden sind.

5. Photochrome Pyrane nach einem der Ansprüche 1 bis 4, wobei innerhalb der Reste R₁ bzw R₂ und/oder innerhalb der Reste R₃ bzw. R₄ jeweils nur einer eine stark elektronenliefernde Gruppe ist

6. Photochrome Pyrane nach Anspruch 5, wobei innerhalb der Reste R₁ bzw. R₂ und innerhalb der Reste R₃ bzw. R₄ jeweils nur einer eine stark elektronenliefernde Gruppe ist.

7. Photochrome Pyrane nach einem der Ansprüche 1 bis 6, wobei die stark elektronenliefernde Gruppe unabhängig voneinander ausgewählt ist aus einer Morpholingruppe, einer Piperidingruppe, einer Azacycloheptangruppe, einer Piperazingruppe, einer Pyrrolidingruppe, einer Pyrazolidingruppe oder einer un-, mono- oder disubstituierten Aminogruppe, wobei die Aminsubstituenten aus einem (C₁-C₆)-Alkylrest, einem (C₃-C₇)-Cycloalkylrest, einem Phenylrest oder einem Benzylrest ausgewählt sein können.

8. Photochrome Pyrane nach einem der vorhergehenden Ansprüche, welche
Spiro-9-fluoren-13'-[6-(N-morpholinyl)-3-[4-(N-morpholinyl)phenyl]-3-phenylindeno[2,1-f]-naphtho[1,2-b]pyran];
Spiro-9-fluoren-13'-[6-dimethylamino-3-[4-(N-morpholinyl)phenyl]-3-phenylindeno[2,1-f]-naphtho[1,2-b]pyran] und
Spiro-9-fluoren-13'-[6,11-bis(N-morpholinyl)-3-[4-(N-morpholinyl)phenyl]-3-phenylindeno[2,1-f]-naphtho[1,2-b]pyran] sind.

9. Verwendung der photochromen Pyrane nach einem der Ansprüche 1 bis 8 in Kunststoffmaterialien.

10. Verwendung nach Anspruch 9, wobei das Kunststoffmaterial eine ophthalmische Unse ist.

## Claims

1. Photochromic pyrans with the general formula (I): 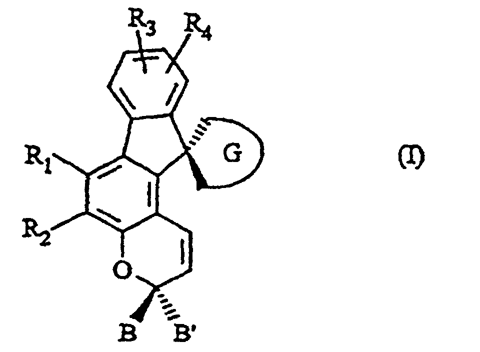 wherein the radicals R₁, R₂, R₃ and R₄ represent respectively independently of each other a substituent, selected from the group α, comprising the sub-groups
A: comprising a hydrogen atom, a (C₁-C₆)alkyl radical, a (C₁-C₆)alkoxy radical, a (C₃-C₇)cycloalkyl radical, which can have one or more heteroatoms, an aryl radical, a heteroaryl radical, a benzyl radical, a hydroxy group, bromine, chlorine and fluorine, and
A': according to which the radicals R₁ and R₂ or R₃ and R₄ form respectively an -O-(CH₂)ₙ-O- group, bonded to the aromatic ring, with n = 1 or 2,
A": comprising strongly electron-attracting groups, selected from -CF₃, -NO₂, -CN and -SO₂R⁵, R⁵ being selected from the sub-group A, and strongly electron-donating groups, selected from a thio(C₁-C₆)alkyl radical, a thiophenyl group, a thiobenzyl group, a thiomorpholine group, a morpholine group, a piperidine group, an azocycloheptane group, a piperazine group, a pyrrolidine group, a pyrazolidine group and an un-, mono- or disubstituted amino group, the amine substituents being able to be selected from a (C₁-C₆)alkyl radical, a (C₃-C₇)cycloalkyl radical, a phenyl radical or a benzyl radical,
with the proviso that R₁ and R₂ are bonded either directly to the benzopyrane unit according to formula (I) or via a thereon anellated aromatic or heteroaromatic ring system, with the formation of a corresponding naphthopyrane unit,
and with the further proviso that, within the radicals R₁ or R₂ and/or within the radicals R₃ or R₄, only one strongly electron-attracting group or strongly electron-donating group respectively is introduced, selected from the sub-group A",
the structural unit G, including the central spiro carbon atom, represents a saturated and/or unsaturated ring member with 5 to 8 carbon atoms, one of which at most can be replaced by a heteroatom, selected from the group, comprising O, S and NR₅, the radical R₅, being defined as above, at least one aromatic or heteroaromatic ring system being anellated on the ring member, the ring system being selected from the group E, comprising benzene, naphthalene, phenanthrene, pyridine, quinoline, furane, thiophene, pyrrole, benzofurane, benzothiophene, indole and carbazole, and the ring system being able to have one or more substituents from the group α;
B and B' being selected independently of each other from one of the following groups a), b), c) or d), wherein they
a) are mono-, di-, and trisubstituted aryl radicals, the aryl radical being phenyl or naphthyl;
b) are unsubstituted, mono- and disubstituted heteroaryl radicals, the heteroaryl radical being pyridyl, furanyl, benzofuran-2-yl, benzofuran-3-yl, thienyl, benzothien-2-yl, benzothien-3-yl or julolidinyl;
the substituents of the aryl or heteroaryl radicals in a) and b) being those selected from a group, comprising hydroxy, amino, mono-(C₁-C₆)alkylamino, di-(C₁-C₆)alkylamino, on the aromatic un-, mono- or disubstituted mono- and diphenylamino, piperidinyl, morpholinyl, carbazolyl, un-, mono- and disubstituted pyrryl, (C₁-C₆)alkyl, (C₁-C₆)alkoxy, bromine, chlorine and fluorine, the aforementioned aromatic and heteroaromatic ring systems being able to be substituted with (C₁-C₆)alkyl, (C₁-C₆)alkoxy, bromine, chlorine and fluorine,
c) are structural units with the following formulae (V) and (W): 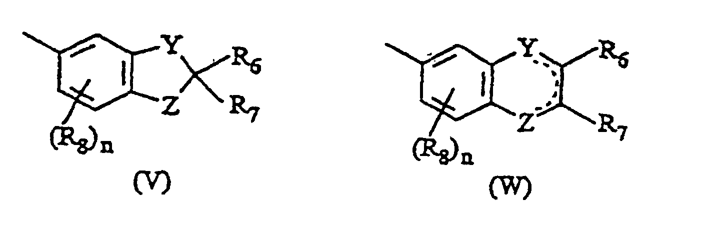 wherein
Y and Z independently of each other are O, S, CH, CH₂ or NR₉, the radical R₉ being selected from the group D, comprising (C₁-C₆)alkyl, (C₁-C₆)acyl, phenyl and hydrogen, the radicals R₆ and R₇ independently of each other represent hydrogen and/or a (C₁-C₆)alkyl radical and the radical R₈ is a substituent from the sub-group A, n being 0, 1, 2 or 3, with the proviso that, if Y in formula (V) is NR₉, Z is carbon,
or
d) B and B' together form an un- mono- or disubstituted fluoren-9-ylide radical or a saturated hydrocarbon radical, which is C₃-C₁₂ spiro-monocyclic, C₇-C₁₂ spiro-bicyclic and/or C₇-C₁₂ spiro-tricyclic, the fluorene substituents being selected from the sub-group A.

2. Photochromic pyrans according to claim 1, the structural unit G in formula (I), including the central spiro carbon atom, being a substituted or unsubstituted fluorene, xanthene, thioxanthene, phenanthrenyl or dihydroanthracene unit.

3. Photochromic pyrans according to claim 1 or 2 which have the general formula (II): 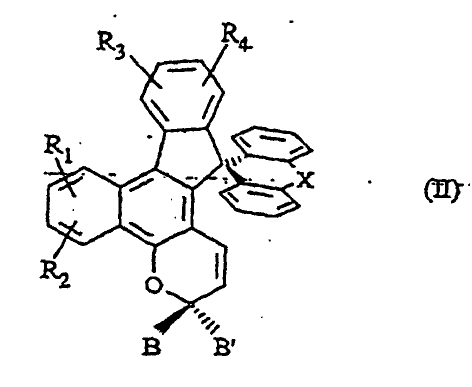 wherein R₁, R₂, R₃ and R₄ are defined as above and X stands for -CR'₂-, -CR'=CR'-, -O-, -S- or an σ single bond, the radical R' being selected from the sub-group A independently of each other, with the above proviso that, within the radicals R₁ or R₂ and/or within the radicals R₃ or R₄ respectively, only one is a strongly electron-attracting group or strongly electron-donating group, selected from the sub-group A".

4. Photochromic pyrans according to claim 3, the radicals R₁ or R₂, being bonded in 6- or 7-position of the naphthopyran unit according to the above formula (II).

5. Photochromic pyrans according to one of the claims 1 to 4, only one being a strongly electron-donating group within the radicals R₁ or R₂, and/or within the radicals R₃ or R₄ respectively.

6. Photochromic pyrans according to claim 5, only one being a strongly electron-donating group within the radicals R₁ or R₂ and within the radicals R₃ or R₄ respectively.

7. Photochromic pyrans according to one of the claims 1 to 6, the strongly electron-donating group being selected independently of each other from a morpholine group, a piperidine group, an azocycloheptane group, a piperazine group, a pyrrolidine group, a pyrazolidine group or from an un-, mono- or disubstituted amino group, the amine substituents being able to be selected from a (C₁-C₆)alkyl radical, a (C₃-C₇)cycloalkyl radical, a phenyl radical or benzyl radical.

8. Photochromic pyrans according to one of the preceding claims, which are
spiro-9-fluorene-13'-[6-(N-morpholinyl)-3-[4-(N-morpholinyl)phenyl]-3-phenyl-indeno[2,1-f]-naphtho[1,2-b]pyran];
spiro-9-fluorene-13'-[6-dimethylamino-3-[4-(N-morpholinyl)phenyl]-3-phenyl-indeno[2,1-f]-naphtho[1,2-b]pyran] and
spiro-9-fluorene-13'-[6,11-bis-(N-morpholinyl)-3-[4-(N-morpholinyl)phenyl]-3-phenyl-indeno[2,1-f]-naptho[1,2-b]pyran].

9. Use of the photochromic pyrans according to one of the claims 1 to 8 in plastic materials.

10. Use according to claim 9, the plastic material being an opthalmic lens.

## Revendications

1. Pyranes photochromiques de formule générale (I) : 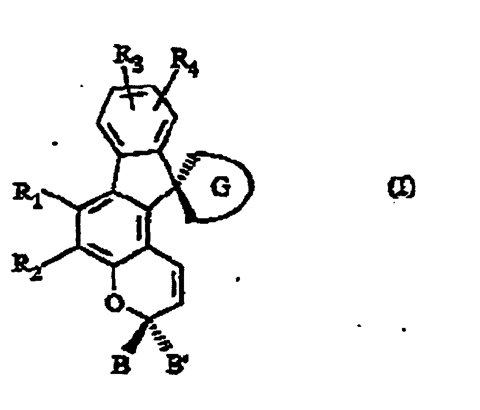 dans laquelle les radicaux R₁, R₂, R₃ et R₄, respectivement indépendamment les uns des autres, représentent un substituant choisi dans le groupe α, constitué des sous-groupes :
A : constitué d'un atome d'hydrogène, d'un radical alkyle en (C₁-C₆), d'un radical alcoxy en (C₁-C₆), d'un radical cycloalkyle en (C₃-C₇), qui peut présenter un ou plusieurs hétéroatomes, d'un radical aryle, d'un radical hétéroaryle, d'un radical benzyle, d'un groupe hydroxy, du brome, du chlore et du fluor ; et
A' : d'après lequel les radicaux R₁ et R₂ ou R₃ et R₄ forment respectivement un groupe -O-(CH₂)ₙ-O- lié à un cycle aromatique, avec n = 1 ou 2,
A'' : constitué de groupes fortement accepteurs d'électrons, choisis parmi -CF₃, -NO₂, -CN et -SO₂R₅, dans laquelle R₅ est choisi dans le sous-groupe A, et de groupes fortement donneurs d'électrons, choisis parmi un radical thio(C₁-C₆)alkyle, un groupe thiophényle, un groupe thiobenzyle, un groupe thiomorpholine, un groupe morpholine, un groupe pipéridine, un groupe azacycloheptane, un groupe pipérazine, un groupe pyrrolidine, un groupe pyrazolidine et un groupe amino non substitué, mono- ou disubstitué, les substituants amine pouvant être choisis parmi un radical alkyle en (C₁-C₆), un radical cycloalkyle en (C₃-C₇), un radical phényle ou un radical benzyle,
à la condition que R₁ et R₂ soient liés soit directement à une unité benzopyrane selon la formule (I), soit par le biais d'un système cyclique aromatique ou hétéroaromatique qui y est condensé, en formant une unité de naphtopyrane correspondante,
et à l'autre condition que, à l'intérieur des radicaux R₁ ou R₂ et/ou à l'intérieur des radicaux R₃ ou R₄, un seul groupe fortement accepteur d'électrons ou groupe fortement donneur d'électrons, choisi dans le sous-groupe A'', soit respectivement introduit,
l'unité structurelle G représente, en intégrant l'atome de carbone spirannique central, un membre cyclique saturé et/ou insaturé, ayant de 5 à 8 atomes de carbone, dont au maximum un peut être remplacé par un hétéroatome, choisi dans le groupe constitué de O, S et NR₅, dans laquelle R₅ est tel que défini précédemment, dans laquelle au moins un système cyclique aromatique ou hétéroaromatique est condensé au membre cyclique, le système cyclique étant choisi dans le groupe E constitué du benzène, du naphtalène, du phénanthrène, de la pyridine, de la quinoline, du furane, du thiophène, du pyrrol, du benzofurane, du benzothiophène, de l'indol et du carbazol, et le système cyclique pouvant présenter un ou plusieurs substituants provenant du groupe α ; B et B', indépendamment l'un de l'autre, sont choisis dans l'un des groupes suivants a), b), c) ou d), qui sont
a) des radicaux aryle mono-, di- et trisubstitués, dans lesquels le radical alkyle est phényle ou naphtyle ;
b) des radicaux hétéroaryle non substitués, mono- et disubstitués, dans lesquels le radical hétéroaryle est pyridyle, furanyle, benzofuran-2-yle, benzofuran-3-yle, thiényle, benzothièn-2-yle, benzothièn-3-yle ou julolidinyle ;
dans lesquels les substituants des radicaux aryle ou hétéroaryle dans a) et b) sont ceux choisis dans un groupe constitué des groupes hydroxy, amino, mono-(C₁-C₆)-alkylamino, di-(C₁-C₆)-alkylamino, mono- et diphénylamino non substitués, mono- ou disubstitués sur l'aromate, pipéridinyle, morpholinyle, carbazolyle, pyrryle non substitué, mono- et disubstitué, alkyle en (C₁-C₆), alcoxy en (C₁-C₆), brome, chlore et fluor, dans lesquels les systèmes cycliques aromatiques et hétéroaromatiques précités peuvent être substitués par des groupes alkyle en (C₁-C₆), alcoxy en (C₁-C₆), brome, chlore et fluor ;
c) des unités structurelles ayant les formules suivantes (V) et (W) : 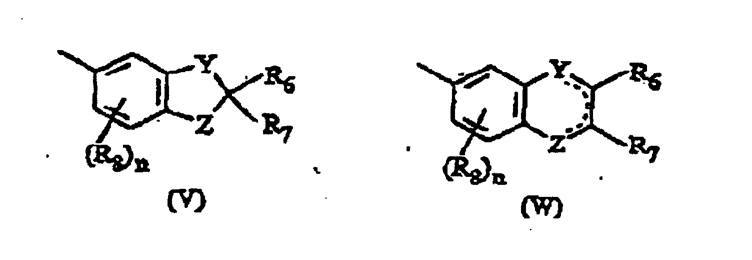 dans lesquelles Y et Z, indépendamment l'un de l'autre, sont O, S, CH, CH₂ ou NR₉, dans laquelle le radical R₉ est choisi dans le groupe D, constitué des groupes alkyle en (C₁-C₆), acyle en (C₁-C₆), phényle et hydrogène, les radicaux R₆ et R₇, indépendamment l'un de l'autre, représentent un hydrogène et/ou un radical alkyle en (C₁-C₆) et le radical R₈ est un substituant du sous-groupe A,
dans lequel n est égal à 0, 1, 2 ou 3, à la condition que, quand Y est NR₉ dans la formule (V), Z est le carbone,
ou
d) B et B' forment, ensemble, un radical fluorèn-9-ylidène non substitué, mono ou di-substitué ou un radical hydrocarbure saturé, qui est spiromonocyclique en C₃-C₁₂, spirobicyclique en C₇-C₁₂ et/ou spirotricyclique en C₇-C₁₂,
dans lesquels les substituants fluorène sont choisis dans le sous-groupe A.

2. Pyranes photochromiques, selon la revendication 1, dans lesquels l'unité structurelle G dans la formule (I), en intégrant l'atome de carbone spirannique central, est une unité fluorène, xanthène, thioxanthène, phénanthrènyle ou dihydroanthracène, substituée ou non substituée.

3. Pyranes photochromiques, selon la revendication 1 ou 2, qui présentent la formule générale (II) : 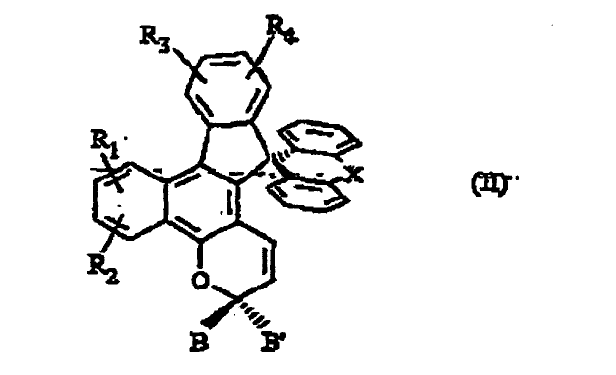 dans laquelle R₁, R₂, R₃ et R₄, sont tels que définis précédemment et X représente -CR'₂-, -CR'=CR'-, -O-, -S- ou une simple liaison σ, dans lesquelles le radical R', indépendamment, est choisi dans le sous-groupe A, à la condition précédente que, à l'intérieur des radicaux R₁ ou R₂ et/ou à l'intérieur des radicaux R₃ ou R₄, respectivement, un seul soit un groupe fortement accepteur d'électrons ou groupe fortement donneur d'électrons, choisi dans le sous-groupe A''.

4. Pyranes photochromiques selon la revendication 3, dans lesquels les radicaux R₁ ou R₂ sont liés en position 6 ou 7 de l'unité naphtopyrane selon la formule précédente (II).

5. Pyranes photochromiques selon l'une des revendications 1 à 4, dans lesquels à l'intérieur des radicaux R₁ ou R₂ et/ou à l'intérieur des radicaux R₃ ou R₄, respectivement, un seul est un groupe fortement donneur d'électrons.

6. Pyranes photochromiques selon la revendication 5, dans lesquels à l'intérieur des radicaux R₁ ou R₂ et/ou à l'intérieur des radicaux R₃ ou R₄, respectivement, un seul est un groupe fortement donneur d'électrons.

7. Pyranes photochromiques selon l'une des revendications 1 à 6, dans lesquels le groupe fortement donneur d'électrons, indépendamment l'un de l'autre, est choisi dans un groupe morpholine, pipéridine, azacycloheptane, pipérazine, pyrrolidine, pyrazolidine ou un groupe amino non substitué, mono- ou disubstitué, dans lequel les substituants amino peuvent être choisis parmi un radical alkyle en (C₁-C₆), un radical cycloalkyle en (C₃-C₇), un radical phényle ou un radical benzyle.

8. Pyranes photochromiques selon l'une quelconque des revendications précédentes, qui sont le
spiro-9-fluorèn-13'-[6-(N-morpholinyl)-3-[4-(N-morpholinyl)phényl]-3-phényl-indèno[2,1-f]-naphto[1,2-b]pyrane]
spiro-9-fluorèn-13'-[6-diméthylamino-3-[4-(N-morpholinyl)phényl]-3-phényl-indèno[2,1-f]-naphto[1,2-b]pyrane] et
spiro-9-fluorèn-13'-[6,11-bis(N-morpholinyl)-3-[4-(N-morpholinyl)phényl]-3-phényl-indèno[2,1-f]-naphto[1,2-b]pyrane].

9. Utilisation des pyranes photochromiques selon l'une des revendications 1 à 8 dans des matières plastiques.

10. Utilisation selon la revendication 9, dans laquelle la matière plastique est une lentille ophtalmique.
